## Europäisches Patentamt
## European Patent Office
## Office européen des brevets

(11) Veröffentlichungsnummer: **0 299 092**
**A1**

(12) **EUROPÄISCHE PATENTANMELDUNG**
Veröffentlicht nach Art. 158 Abs. 3 EPÜ

(21) Anmeldenummer: 88901668.9

(22) Anmeldetag: 07.01.88

Daten der zugrundeliegenden internationalen Anmeldung:

(86) Internationale Anmeldenummer:
PCT/SU88/00006

(87) Internationale Veröffentlichungsnummer:
WO88/05794 (11.08.88 88/18)

(51) Int. Cl.³: **C 08 F 220/56**
C 08 F 267/10, C 12 N 1/00

(30) Priorität: 30.01.87 SU 4182247
25.06.87 SU 4278310

(43) Veröffentlichungstag der Anmeldung:
18.01.89 Patentblatt 89/3

(84) Benannte Vertragsstaaten:
AT BE CH DE FR GB IT LI LU NL SE

(71) Anmelder: KIEVSKY MEDITSINSKY INSTITUT IMENI
AKADEMIA A.A.BOGOMOLTSA
Bulvar T.G.Shevchenko, 13
Kiev, 252004(SU)

(72) Erfinder: GASHINSKY, Vladimir Vladislavovich
ul. Repina, 7/13-11
Kiev, 252004(SU)

(72) Erfinder: SHIROBOKOV, Vladimir Pavlovich
ul. Malyshko, 3-538
Kiev, 252192(SU)

(72) Erfinder: MARCHENKO, Nikolai Nikolaevich
bulvar I.Lepse, 49-33
Kiev, 252065(SU)

(72) Erfinder: VOITSEKHOVSKY, Valery Grigorievich
ul. Betkhovena, 18
Kiev, 252170(SU)

(72) Erfinder: KRAINJUKOVA, Tatyana Ivanovna
ul. Zakrevskogo, 11-120
Kiev, 252222(SU)

(72) Erfinder: ONISCHENKO, Vladimir Viktorvich
ul. Novaya, 6-140 Kievskaya obl.
Fastov, 255530(SU)

(74) Vertreter: von Füner, Alexander, Dr. et al,
Patentanwälte v. Füner, Ebbinghaus, Finck Mariahilfplatz
2 & 3 Postfach 95 01 60
D-8000 München 95(DE)

(54) **VERFAHREN ZUR HERSTELLUNG EINES FESTEN NÄHRMEDIUMS ZUR KULTIVIERUNG VON MIKROORGANISMEN.**

(57) Verfahren zur Herstellung der Plattenkultur für die Kultivierung der Mikroorganismen besteht in der Kopolymerisation von Akrylamid mit N,N'-Methylenbisakrylamid in Gegenwart des verweigten Polyakrylamides mit einer durchschnittlichen Molekularmasse von $7,2.10^4$ bei deren Massenverhältnis von 7,0-15,0:0,11-0,21 bzw. 0,028-0,11 und eines Aktivators in der physiologischen Lösung bis zur Bildung eines Gels, im Abwaschen und dessen Imprägnieren mit dem Nährsubstrat.

Croydon Printing Company Ltd.

EP 0 299 092 A1

# VERFAHREN ZUR HERSTELLUNG DER PLATTENKULTUR FÜR DIE KULTIVIERUNG DER MIKROORGANISMEN

## Gebiet der Technik

Die vorliegende Erfindung bezieht sich auf ein Verfahren zur Herstellung der Plattenkultur für die Kultivierung der Mikroorganismen auf Grundlage des Polyakrylamidgels.

## Zugrundeliegender Stand der Technik

Es ist ein Verfahren zur Herstellung der Plattenkultur für die Kultivierung der Mikroorganismen durch Kopolymerisation von Akrylamid und N,N' -Methylenbisakrylamid bei deren Massenverhältnis von 15,0-20,0 : 0,019-0,132 in Gegenwart eines Aktivators in der physiologischen Lösung bis zur Bildung eines Gels, durch Abwaschen und dessen Quellung in der physiologischen Lösung innerhalb von 16 bis 20 Stunden bei einer Temperatur von 50 bis 60 $^{o}$C und darauffolgende Imprägnierung mit dem Nährsubstrat bekannt.

Diese Plattenkultur hat eine den Agar-Agarmedien nahe Dichte, ist elastisch und klar. Sie bewirkt einen guten Gleiteffekt der bakteriologischen Öse bei der Impfung und Abnahme der Mikroorganismen ohne Schädigung deren Oberfläche, der durch eine langwierige Quellung (16 bis 20 Stunden) erreicht wird, was den technologischen Prozeß im großen und ganzen wesentlich verlängert.

Es ist ein Verfahren zur Herstellung der Plattenkultur für Kultivierung der Mikroorganismen bekannt, das in der Kopolymerisation von Akrylamid und N,N' -Methylenbisakrylamid bei einem Massenverhältnis von 0,5-40,0:2,5-12 in Gegenwart eines Aktivators in der physiologischen Lösung besteht. Das erhaltene Polyakrylamidgel wird dem Abwaschen und der Imprägnierung mit einem Nährsubstrat ausgesetzt (SU, A, 977466). Die genannte Plattenkultur besitzt eine erhöhte Verletztbarkeit bei der Imprägnierung, Sterilisation und beim Impfen der Mikroorganismen. Die Abnahme einzelner Mikroorganismen arten mit der bakteriologischen Öse von der Oberläche solch eines Nährmediums ist erschwert.

- 2 -

In den beschriebenen Verfahren wird die Kopolymerisation in speziellen Formiereinrichtungen ohne Luftzutritt verwirklicht, um das Auftreten der rauhen Oberfläche beim geformten Polyakrylamidgel auszuschließen, die dessen Ausnutzung bei der Herstellung des Nährmediums vorbeugt. Solch eine Technologie erschwert die Massenproduktion von Nährmedien, beispielsweise in den Petrischalen und in anderen Laborbehältern.

Offenbarung der Erfindung

Der Erfindung liegt die Aufgabe zugrunde, ein Verfahren zur Herstellung der Plattenkultur für die Kultivierung der Mikroorganismen durch Ausnutzung bei der Kopolymerisation eines strukturierenden Mittels zu entwickeln, das dadurch dem Nährmedium die den Agar-Agarmedien nahen Eigenschaften unter einem guten Gleiteffekt der bakteriologischen Öse beim Impfen der Mikroorganismen bewirkt.

Die Aufgabe wird dadurch gelöst, daß ein Verfahren zur Herstellung der Plattenkultur für die Kultivierung der Mikroorganismen vorgeschlagen wird, das in der Kopolymerisation von Akrylamid mit N,N' -Methylenbisakrylamid in Gegenwart eines Aktivators in der physiologischen Lösung bis zur Bildung eines Gels, im Abwaschen und in dessen Imprägnierung mit dem Nährsubstrat besteht, in welchem erfindungsgemäß die Kopolymerisation von Akrylamid mit N,N' -Methylenbisakrylamid in Gegenwart des verzweigten Polyakrylamids mit einer durchschnittlichen Molekularmasse von $7,2.10^{4}$ bei deren Massenverhältnis von $7,0$--$15,0:0,11$-$0,21$ bzw. $0,028$-$0,11$ durchgeführt wird.

Das vorgeschlagene Verfahren sichert eine gute Qualität des Nährbodens bei der vereinfachten Herstellungstechnologie. Die Kopolymerisation läßt sich auf Kosten des strukturierenden Mittels an der Luft ohne Verwendung spezieller Ausrüstungen verwirklichen.

Um die Kopolymerisation zu beschleunigen, ist es wünschenswert, die wäßrige Lösung des genannten verzweigten Polyakrylamids zu verwenden. Es ist zweckmäßig, bei der Imprägnierung des Polyakrylamidgels das Nährsubstrat zu verwenden, welches Polyäthylenoxid in einer Menge von

- 3 -

0,02 bis 0,2 g/l enthält. Das trägt zur Formung solch einer oberflächlichen Monoschicht beim Nährmedium bei, die einen deutlichen ausgeprägten Gleiteffekt der bakteriologischen Öse beim Impfen der Mikroorganismen sichert.

Beste Ausführungsvariante der Erfindung

Die Plattenkultur für die Kultivierung der Mikroorganismen wird erfindungsgemäß wie folgt bereitet.

Man stellt ein Reaktionsgemisch her, das die Ausgangsmonomere, das verzweigte Polyakrylamid mit einer Molekularmasse von $7,2.10^4$ und die Aktivatoren der Kopolymerisation in der physiologischen Lösung enthält. Man kann die wäßrige Lösung des genannten verzweigten Polyakrylamids verwenden. Das Verhältnis von Akrylamid zum verzweigten Polyakrylamid im Reaktionsgemisch beträgt 7,0-1,5 bzw. 0,028-0,11.

Als physiologische Lösung verwendet man eine 0,5%ige oder 0,9%ige Natriumchloridlösung, eine 5%ige wäßrige Glukoselösung, die Lockesche, Hanks'sche, Erlsche Lösungen und andere. Die Kopolymerisation verwirklicht man an der Luft, in der Petrischale oder in anderen aus verschiedenen Stoffen hergestellten Behältern.

Die Durchführung der Kopolymerisation in Gegenwart von verzweigtem Polyakrylamid bei den angegebenen Verhältnissen trägt zur Herstellung des Polyakrylamidgels mit einer der Agargrundlage nahen Dichte bei. Außerdem führt die Verwendung des verweigten Polyakrylamids zu einer zusätzlichen Strukturierung des Polyakrylamidgels, was ermöglicht, dieses mit einer glatten Oberfläche bei der Polymerisation in den Petrischalen an der Luft herzustellen. Früher erreichte man solch einen Effekt nur bei der Durchführung der Polymerisation in speziellen Einrichtungen ohne Luftzutritt unter Ausnutzung von inerten Gasen. Bei der Herstellung von Nährmedien wird erfindungsgemäß die Stufe der Gelquellung innerhalb von 16 bis 20 Stunden ausgeschlossen.

Alle oben aufgezählten Vorteile vereinfachen bedeutend die Herstellungstechnologie des Nährmediums.

Nach dem Abwaschen wird das Polyakrylamidgel der chemischen, Wärme- oder Radiationssterilisation unterzogen. Die Imprägnierung des angegeben Gels mit den Substraten für die Nahrung der Mikroorganismen kann vor und nach der Sterilisation vor sich gehen. Es wird empfohlen, Nährsubstrate auszunutzen, die Polyäthylenoxid mit einer Molekularmasse ab 500 Tausend in einer Menge von 0,02 bis 0,2 g/l enthalten.

Die Zusammensetzung des Substrats wird durch die Bedürfnisse konkreter Gruppen oder Arten von Mikroorganismen und Zellen bestimmt.

Zur Bestimmung der Qualität der Nährmedien sowie zur Untersuchung biologischer Eigenschaften der Mikroorganismen werden routinemäßige Untersuchungsmethoden ausgenutzt.

Nachstehend werden konkrete Beispiele der Verwirklichung des erfindungsgemäßen Verfahrens angeführt.

Beispiel 1.

Zur Gelbereitung werden 5 Lösungen A, B, C, D, E nach folgender Vorschrift bereitgestellt (die Mengen der Komponenten sind pro 100 ml Lösung angegeben).

1. Bereitstellung der Lösung A.

0,5 ml N,N,N', N' -Tetramethyläthylendiamin (TMÄD) werden in 99,5 ml physiologischer Lösung gelöst.

2. Bereitstellung der Lösung B.

Man löst 0,735 g N,N' -Methylenbisakrylamid (MBA) in 50 ml physiologischer Lösung, erwärmt auf eine Temperatur von 60 $^\circ$C, gibt 24,5 g Akrylamid (AA) zu, mischt bis zum vollen Auflösen. Die erhaltene Lösung wird abfiltriert mit der physiologischen Lösung und bis zum Erreichen eines Volumens von 100 ml verdünnt.

3. Bereitstellung der Lösung C.

Man löst 0,2 g Ammoniumpersulfat (AP) in 50 ml physiologischer Lösung und gibt die letztere bis zur Auffüllung auf 100 ml hinzu.

4. Bereitstellung der Lösung D.

Man löst 10,0 g verzweigtes Polyakrylamid (PAA) mit einer Molekularmasse von $7,2 \times 10^4$ in 50 ml physiologischer Lösung bei einer Temperatur von 50 bis 60 $^{o}$C. Man gibt die physiologische Lösung zu, bis das Volumen des Gemisches 100 ml erreicht.

5. Bereitstellung der Lösung E.

Man gibt 0,5 ml der Lösung D zu 99,5 ml der Lösung C hinzu, mischt sorgfältig durch.

Man bereitet das Reaktionsgemisch aus den Lösungen in folgenden Verhältnissen: A:B:E = 1:2:4, was dem Massenverhältnis von Akrylamid, N,N' -Methylenbisakrylamid und dem verweigten Polyakrylamid (7,0:0,21:0,028) entspricht. Man gießt das Gemisch in die Petrischalen ein und läßt an der Luft stehen. Die Kopolymerisation geht innerhalb von 10 bis 15 Minuten bis zur Bildung eines Gels vor sich, das nachher mit der physiologischen Lösung innerhalb von 1 Stunde abgewaschen wird.

Die Plattenkultur wird durch Imprägnierung des erhaltenen Gels mit der tryptischen Hottinger-Bouillon bereitet.

Die Plattenkultur wird mit Staphylococcus aureus beimpft und bei einer Temperatur von 37 $^{o}$C innerhalb von 24 Stunden inkubiert. Die Plattenkultur besitzt gute Adhäsionseigenschaften, ist dicht und klar, elastisch, hat eine glatte Oberfläche, die den Gleiteffekt der bakteriologischen Öse beim Impfen der Mikroorganismen ohne Schädigung der Oberfläche bewirkt. Die gezüchteten Mikroorganismen weisen die dafür kennzeichnenden kulturellen und morphologischen Merkmale auf.

Beispiel 2.

Zur Bereitung des Polyakrylamidgels werden die Lösungen A, B, C, D ebenso wie in Beispiel 1 verwendet.

Bereitstellung der Lösung E.

1,0 ml der Lösung D wird zu 99,0 der Lösung C zugesetzt, sorgfältig durchgemischt.

Das Reaktionsgemisch bereitet man aus den Lösungen bei den Verhältnissen wie A:B:E=1:2:4, was dem Massenverhältnis von Akrylamid, N,N' -Methylenbisakrylamid und dem verzweigten Polyakrylamid (7,0:0,21:0,057) entspricht. Man gießt das Gemisch in die Petrischalen ein und läßt diese für die Polymerisation an der Luft stehen. Die Kopolymerisation geht innerhalb von 5 bis 10 Minuten bis zur Bildung des Gels vor sich, das nachher mit der physiologischen Lösung innerhalb von 1 Stunde abgewaschen wird.

Das erhaltene Gel wird mit der Fleisch-Pepton-Bouillon innerhalb von 3 Stunden bei einer Temperatur von 56 °C imprägniert und anschließend innerhalb von 30 Minuten sterilisiert.

Die Plattenkultur wird mit Escherichia coli beimpft und bei einer Temperatur von 37 °C innerhalb von 24 Stunden inkubiert. Die gezüchteten Mikroorganismen weisen die dafür kennzeichnenden kulturellen und morphologischen Merkmale auf. Die Plattenkultur besitzt gute Adhäsionseigenschaften, ist dicht, klar und elastisch, hat eine glatte Oberfläche, die den Gleiteffekt der bakteriologischen Öse beim Impfen der Mikroorganismen ohne Schädigung der Oberfläche bewirkt.

Beispiel 3.

Zur Herstellung des Polyakrylamidgels bereitet man drei Lösungen A, B, C nach folgender Vorschrift.

1. Bereitstellung der Lösung A.

Man löst 0,23 ml N,N,N'N' -Tetramethyläthylendiamin in 50 ml physiologischer Lösung und gibt die physiologische Lösung bis zur Auffüllung auf 100 ml hinzu.

2. Bereitstellung der Lösung B.

Man löst 0,4 g N,N' -Methylenbisakrylamid in 50 ml physiologischer Lösung, erwärmt auf eine Temperatur von 60 °C, gibt 53 g Akrylamid hinzu, mischt bis zum vollen Auflösen. Die erhaltene Lösung wird abfiltriert und bis zur Auffüllung auf 100 ml mit der physiologischen Lösung verdünnt.

3. Die Lösungen C und D werden ähnlich mit dem Beispiel 1 bereitet.

4. Bereitstellung der Lösung E.

Man gibt 2 ml der Lösung D in 98 ml der Lösung C zu, mischt sorgfältig durch.

Aus den bereiteten Lösungen stellt man das Reaktionsgemisch her. Dazu mischt man die Lösungen in folgenden

- 7 -

Verhältnissen A:B:E=1:2:4 durch, was dem Massenverhältnis von Akrylamid, N,N'-Methylenbisakrylamid und dem verzweigten Polyakrylamid (15,0:0,11:0,11) entspricht, und gießt das Gemisch in die Petrischalen ein. Die Kopolymerisation führt man an der Luft innerhalb von 10 bis 15 Minuten bis zur Bildung eines Gels durch. Das erhaltene Gel wäscht man mit der physiologischen Lösung innerhalb von 1 Stunde ab, imprägniert mit der Fleisch--Pepton-Bouillon innerhalb von 3 Stunden bei einer Temperatur von 50 bis 60 $^{o}$C, beimpft nach der Sterilisation mit der Sporenkultur.

Der erhaltene Plattenkultur weist gute Adhäsionseigenschaften auf, ist elastisch, dicht, durchsichtig, hat eine glatte Oberfläche, einen deutlich ausgeprägten Gleiteffekt beim Impfen der Mikroorganismen mit der bakteriologischen Öse. Die Kultur weist typische Mekrmale auf.

Beispiel 4.

Das Polyakrylamidgel wird ähnlich mit dem Beispiel 1 hergestellt und die Imprägnierung wird in der tryptischen Hottinger-Bouillon, die 0,2 g/l Polyäthylenoxyd enthält, innerhalb von 3 Stunden bei einer Temperatur von 56 $^{o}$C durchgeführt. Das imprägnierte Gel wird innerhalb von 30 Minuten sterilisiert und mit Staphylococcus aureus beimpft.

Die Plattenkultur besitzt gute Adhäsionseigenschaften, ist elastisch, dicht, durchsichtig, weist einen deutlichen Gleiteffekt beim Impfen der Mikroorganismen mit der bakteriologischen Öse ohne Schädigung der Oberfläche auf. Die Kultur hat typische Merkmale.

Beispiel 5.

Das Polyakrylamidgel wird ähnlich mit dem Beispiel 3 hergestellt und die Imprägnierung wird mit der Fleisch--Pepton-Bouillon, die 0,02 g/l Polyäthylenoxid enthält, innerhalb von 3 Stunden bei einer Temperatur von 56 $^{o}$C durchgeführt und anschließend das Gel wird innerhalb von 30 Minuten sterilisiert.

Die erhaltene Plattenkultur besitzt gute Adhäsions-

- 8 -

eigenschaften, ist elastisch, dicht, durchsichlig, weist einen deutlichen Gleiteffekt der bakteriologischen Öse beim Impfen der Mikroorganismen ohne Schädigung der Oberfläche des Mediums auf.

Als Fest-Mikroorganismus wird Escherichia coli ausgenutzt.

Die Kultur wächst in Form von typischen Kolonien.

Industrielle Anwendbarkeit

Die erfindungsgemäße Plattenkultur findet ihre Anwendung in der Medizin und Biotechnologie

- 9 -

## PATENTANSPRÜCHE

1. Verfahren zur Herstellung der Plattenkultur für die Kultivierung der Mikroorganismen, das in der Kopolymerisation von Akrylamid mit N,N'-Methylenbisakrylamid in Gegenwart eines Aktivators in der physiologischen Lösung bis zur Bildung eines Gels, im Abwaschen und in dessen Imprägnieren mit dem Nährsubstrat besteht, d a d u r c h  g e k e n n z e i c h n e t, daß die Kopolymerisation von Akrylamid mit N,N' -Methylenbisakrylamid in Gegenwart des verzweigten Polyakrylamids mit einer durchschnittlichen Molekularmasse von $7,2.10^4$ bei deren Massenverhältnis von 7,0-15,0:0,11-0,21 bzw. 0,028-1,11 durchgeführt wird.

2. Verfahren nach Anspruch 1, d a d u r c h  g e - k e n n z e i c h n e t, daß die wäßrige Lösung des angegebenen Polyakrylamids verwendet wird.

3. Verfahren nach Anspruch 1 bis 2, d a d u r c h  g e k e n n z e i c h n e t, daß die Imprägnierung des genannten Gels mit einem Nährsubstrat verwirklicht wird, das Polyäthylenoxid in einer Menge von 0,02 bis 0,2 g/l enthält.

## I. CLASSIFICATION OF SUBJECT MATTER (if several classification symbols apply, indicate all) 6

According to International Patent Classification (IPC) or to both National Classification and IPC

IPC$^4$ C 08 F 220/56, 267/10, C 12 N 1/00

## II. FIELDS SEARCHED

### Minimum Documentation Searched 7

| Classification System | Classification Symbols |
|---|---|
| IPC$^4$ | C 08 F 220/56, 267/10, C 12 N 1/00 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | SU, A1, 977466, (Kievsky ordena Trudovogo Krasnogo Znameni meditsinsky institut im. akad. A.A. Bogomoltsa), 30 November 1982 (30.11.82), see columns 5,6 (cited in the description) | 1-3 |
| A | SU, A1, 1213069, (Institut mikrobiologii AN SSSR i Ordena Lenina institut elementoorganicheskikh soedineny), 23 February 1986 (23.02.86), see the abstract | 3 |
| A | DE, A1, 3106456, (Röhm GmbH), 7 October 1982 (07.10.82), see the abstract, example 11 | 1-2 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 14 March 1988 (14.03.88) | 28 April 1988 (28.04.88) |

| International Searching Authority | Signature of Authorized Officer |
|---|---|
| ISA/SU | |

Form PCT/ISA/210 (second sheet) (January 1985)